# EUROPEAN PATENT APPLICATION

(11) **EP 2 345 654 A1**
(43) Date of publication of application: **20.07.2011**
(21) Application number: 10150102.1
(22) Date of filing: 05.01.2010
(51) Int. Cl.: C07D 487/04, A61P 25/20, A61K 31/437

(54) **Eszopiclone particles and a process for their preparation**

(71) Applicant: LEK Pharmaceuticals d.d., 1526 Ljubljana (SI)
(72) Inventor: The designation of the inventor has not yet been filed

(57) **Abstract**

The present invention provides crystalline eszopiclone having particles in the shape of sticks that enables process for the preparation of pharmaceutical composition without segregation of drug substance. Further, the present invention provides the process for preparing crystalline eszopiclone having particles in the shape of sticks.

## Description

### Field of the invention

The present invention relates to a crystalline eszopiclone with particles with particular shapes and a process for their preparation.

### Description of the background art

Eszopiclone or S-zopiclone is a cyclopyrrolone drug with sedative and hypnotic properties. The chemical name of eszopiclone is (+)-5(S)-6-(chloropyridin-2-yl)-7-oxo-6,7-dihydro-5H-pyrrolo[3,4-b]pyrazin-5-yl 4-methylpiperazine-1-carboxylate.

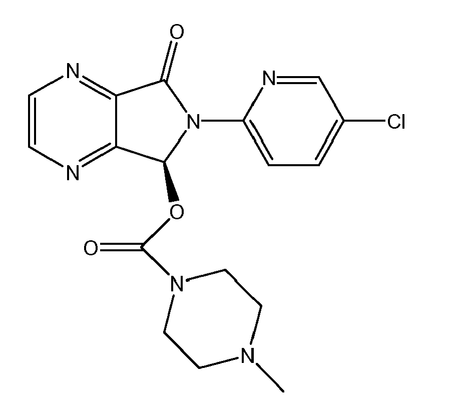

Eszopiclone was first disclosed in EP 495717 and was recrystallized from acetonitrile. A commercially available eszopiclone containing product is Lunesta ®.

Crystallization of eszopiclone from different organic solvents are described in several prior art documents, e.g. US 6319926, Chirality, 1993, 5, 419, WO 00/69442, US 2007/0054914 A1, WO 2007/083188, WO 2008/002629 A1. However, according to the processes described in above prior art a cooling of eszopiclone solution in organic solvents without stirring was performed. Only a stirring of already cooled solution comprising precipitated eszopiclone was performed in processes described in the following patent applications WO 2007/124025, US 2007/0098788 A1, WO 2008/126105 A2, WO 2009/101634 A2.

### Summary of the invention

The present invention provides the following aspects, subject matters and preferred embodiments, which respectively taken alone or in combination, contribute to solving the object of the present invention:
(1) A crystalline eszopiclone having particles with average ratio of width and length between 1:2 and 1:15 and average ratio of thickness and width between 1:1 and 1:5, preferably particles with average ratio of width and length between 1:2 and 1:10 and average ratio of thickness and width between 1:1 and 1:4, more preferably particles with average ratio of width and length between 1:4 and 1:8 and average ratio of thickness and width between 1:1 and 1:3, even more preferably particles with average ratio of width and length between 1:5 and 1:7 and average ratio of thickness and width between 1:1 and 1:3, the most preferably particles with average ratio of width and length 1:6 and average ratio of thickness and width between 1:1 and 1:3.
(2) A crystalline eszopiclone according to item (1) having a particle size distribution as determined by volume by laser-diffraction method of d(0.9) is between 200 µm and 800 µm.
(3) A crystalline eszopiclone according to items (1) or (2) having a particle size distribution as determined by volume by laser-diffraction method of d(0.9) is between 200 µm and 800 µm, preferably 300 µm and 600 µm, more preferably 300 µm and 500 µm, most preferably 350 µm and 450 µm.
(4) A crystalline eszopiclone according to item (3) having a particle size distribution as determined by volume by laser-diffraction method of d(0.5) is between 100 µm and 250 µm.
(5) A crystalline eszopiclone according to item (1) having a particle size distribution as determined by volume by laser-diffraction method of:
   a) d(0.9) is between 600 µm and 800 µm and d(0.5) is between 300 µm and 500 µm, or
   b) d(0.9) is between 400 µm and 600 µm and d(0.5) is between 200 µm and 350 µm, or
   c) d(0.9) is between 350 µm and 450 µm, and d(0.5) is between 100 µm and 250 µm, or
   d) d(0.9) is between 200 µm and 350 µm, and d(0.5) is between 100 µm and 150 µm, preferably, d(0.9) is between 350 µm and 450 µm, and d(0.5) is between 100 µm and 250 µm.
(6) A process for preparing a crystalline eszopiclone according to any one of items (1) to (5) comprising the following steps:
   a) mixing eszopiclone with an organic solvent to obtain a slurry,
   b) heating the slurry of step a) to reflux to obtain a clear solution,
   c) cooling the solution under stirring at 50 rpm to 200 rpm, to obtain precipitate,
   d) recovering eszopiclone particles,
      wherein an organic solvent is selected from the group consisting of C₁-C₆ alcohols, C₁-C₆ alkyl acetates, C₁-C₆ alkyl ketones and a mixture of C₁-C₆ alcohols and acetone, preferably from the group consisting of ethanol, 1-propanol, 2-propanol, ethyl acetate, isopropyl acetate, isobutyl acetate, methyl ethyl ketone, methyl isobutyl ketone and a mixture of methanol and acetone, more preferably from 2-propanol and ethyl acetate.
(7) A process for preparing a crystalline eszopiclone comprising the following steps:
   a) mixing eszopiclone with an organic solvent to obtain a slurry,
   b) heating the slurry of step a) to reflux to obtain a clear solution,
   c) cooling the solution under stirring at 50 rpm to 200 rpm, preferably at 80 rpm to 170 rpm, more preferably at 100 rpm to 170 rpm, most preferably at 150 rpm, to obtain precipitate,
   d) recovering eszopiclone particles,
      wherein an organic solvent is selected from the group consisting of C₁-C₆ alcohols, C₁-C₆ alkyl acetates, C₁-C₆ alkyl ketones and a mixture of C₁-C₆ alcohols and acetone, preferably from the group consisting of ethanol, 1-propanol, 2-propanol, ethyl acetate, isopropyl acetate, isobutyl acetate, methyl ethyl ketone, methyl isobutyl ketone and a mixture of methanol and acetone, more preferably, selected from 2-propanol and ethyl acetate.
(8) A process according to items (6) or (7), wherein seeding of hot eszopiclone solution is performed after step b).
(9) A process according to any one of items (6) to (8), wherein a cooling in step c) is gradually cooling to the temperature of 0 °C to 5 °C over 2 hour to 20 hours, preferably over 4 hours to 14 hours, more preferably over 8 hours.
(10) A process according to any one of items (6) to (8), wherein a cooling in step c) is a two stage cooling comprising the following sub-steps:
   c1) gradually cooling to the temperature of 20 °C to 25 °C over 1 hour to 10 hours,
   c2) gradually cooling to the temperature of 0 °C to 5 °C over 1 hour to 10 hours.
(11) A process according to any one of items (6) to (10), wherein precipitate obtained in step c) is stirred at 50 rpm to 200 rpm, preferably at 80 rpm to 170 rpm, more preferably at 100 rpm to 170 rpm, most preferably at 150 rpm, for additional 1 hour to 3 hours, preferably for additional 2 hours.
(12) Use of a crystalline eszopiclone according to any one of items (1) to (5) for the preparation of pharmaceutical composition.
(13) A pharmaceutical composition comprising a crystalline eszopiclone according to any one of items (1) to (5).
(14) Use of the pharmaceutical composition according to item (13) for the preparation of a medicament with sedative and hypnotic properties.

### Detailed description of the invention

The solid state physical properties of an active pharmaceutical ingredient (API), such as eszopiclone, can be very important in formulating a drug substance and are directly related to the easiness and reproducibility of formulation.

Shape of the particles, for example may effect flowability and mixability of a drug substance.

Precipitation of eszopiclone from solvents like ethanol, 2-propanol, 2-butanol, ethyl acetate, tetrahydrofuran, a mixture of 2-propanol and ethyl acetate without stirring during the cooling of eszopiclone solution, as described in prior art, resulted in particles having shape of tiles or slumps which tended to segregate during the preparation of pharmaceutical composition.

Therefore, it is essential to ensure eszopiclone having particles in the right shape in order to prevent segregation of particles and a process for the preparation thereof.

We have surprisingly found that when moderate stirring was applied during the cooling in the process of the precipitation of eszopiclone from organic solvents, e.g. 2-propanol or ethyl acetate, a crystalline eszopiclone with particles in the shape of sticks was formed. Obtained particles Particles, obtained in the shape of sticks, enable process for the preparation of pharmaceutical composition without segregation of drug substance.

A crystalline eszopiclone having particles in the shape of sticks allows preparation of pharmaceutical composition having equally distributed drug substance.

The present invention provides a crystalline eszopiclone having particles in the shape of sticks that enables process for the preparation of pharmaceutical composition without segregation of drug substance.

The term "particles in the shape of stick" means particles with average ratio of width and length between 1:2 and 1:15 and average ratio of thickness and width between 1:1 and 1:5, preferably particles with average ratio of width and length between 1:2 and 1:10 and average ratio of thickness and width between 1:1 and 1:4, more preferably particles with average ratio of width and length between 1:4 and 1:8 and average ratio of thickness and width between 1:1 and 1:3, even more preferably particles with average ratio of width and length between 1:5 and 1:7 and average ratio of thickness and width between 1:1 and 1:3, the most preferably particles with average ratio of width and length 1:6 and average ratio of thickness and width between 1:1 and 1:3.

Particle shape of eszopiclone was characterized by scanning electron microscopy (SEM).

A first object of the present invention is a crystalline eszopiclone having particles with average ratio of width and length between 1:2 and 1:15 and average ratio of thickness and width between 1:1 and 1:5, preferably particles with average ratio of width and length between 1:2 and 1:10 and average ratio of thickness and width between 1:1 and 1:4, more preferably particles with average ratio of width and length between 1:4 and 1:8 and average ratio of thickness and width between 1:1 and 1:3, even more preferably particles with average ratio of width and length between 1:5 and 1:7 and average ratio of thickness and width between 1:1 and 1:3, the most preferably particles with average ratio of width and length 1:6 and average ratio of thickness and width between 1:1 and 1:3.

In a preferred embodiment of the present invention a crystalline eszopiclone having particles in the shape of sticks having a particle size distribution as determined by volume by laser-diffraction method of d(0.9) is between 200 µm and 800 µm, preferably 300 µm and 600 µm, more preferably 300 µm and 500 µm, most preferably 350 µm and 450 µm.

In a preferred embodiment of the present invention a crystalline eszopiclone having particles in the shape of sticks having a particle size distribution as determined by volume by laser-diffraction method of:
a) d(0.9) is between 600 µm and 800 µm and d(0.5) is between 300 µm and 500 µm, or
b) d(0.9) is between 400 µm and 600 µm and d(0.5) is between 200 µm and 350 µm, or
c) d(0.9) is between 350 µm and 450 µm, and d(0.5) is between 100 µm and 250 µm, or
d) d(0.9) is between 200 µm and 350 µm, and d(0.5) is between 100 µm and 150 µm, preferably, d(0.9) is between 350 µm and 450 µm, and d(0.5) is between 100 µm and 250 µm.

A particle size distribution of d(0.9) as used herein is defined as the distribution where 90 volume percent of the particles are smaller than that size given. A particle size distribution of d(0.5) as used herein is defined as the distribution where 50 volume percent of the particles are smaller than that size given.

Another object of the present invention is a process for preparing a crystalline eszopiclone having particles in the shape of sticks comprising the following steps:
a) mixing eszopiclone with an organic solvent to obtain a slurry,
b) heating the slurry of step a) to reflux to obtain a clear solution,
c) cooling the solution under moderate stirring to obtain precipitate,
d) recovering eszopiclone having particles in the shape of sticks.

Solvents used in the step a) are selected from group consisting of C₁-C₆ alcohols, preferably ethanol, 1-propanol, and 2-propanol, C₁-C₆ alkyl acetates, preferably ethyl acetate, isopropyl acetate and isobutyl acetate, C₁-C₆ alkyl ketones, preferably methyl ethyl ketone and methyl isobutyl ketone and a mixture of C₁-C₆ alcohols and acetone, preferably methanol and acetone. Preferably, solvents used in step a) are selected from group consisting of ethanol, 1-propanol, 2-propanol, ethyl acetate, isopropyl acetate, isobutyl acetate, methyl ethyl ketone, methyl isobutyl ketone and a mixture of methanol and acetone. More preferably, solvents used in step a) are 2-propanol and ethyl acetate.

The solution in step b) can be filtered, to remove foreign particles, while maintaining the temperature of the filtered solution and filtrate in the range to ensure that eszopiclone remains dissolved in the solution.

Optionally, seeding of hot eszopiclone solution can be performed after step b).

Moderate stirring in the present invention means stirring at 50 rpm to 200 rpm, preferably at 80 rpm to 170 rpm, more preferably at 100 rpm to 170 rpm, most preferably at 150 rpm. Stirring may be performed for example by four-pitch blade stirrer.

In one option the solution of the step b) is gradually cooled under moderate stirring to the temperature of about 0 °C to 5 °C over about 2 hour to about 20 hours, preferably over about 4 hours to about 14 hours, more preferably over about 8 hours. Optionally, the mixture at the temperature of about 0 °C to 5 °C is further stirred for additional 1 hour to 3 hours, preferably for additional 2 hours under moderate stirring.

In another option the solution of the step b) is gradually cooled under moderate stirring, firstly to the temperature of about 20 °C to 25 °C over about 1 hour to about 10 hours, preferably over about 2 hours to about 7 hours, more preferably over about 4 hours, following to the temperature of about 5 °C to 0 °C over about 1 hour to about 10 hours, preferably over about 2 hours to about 7 hours, more preferably over about 4 hours. Optionally, the mixture at the temperature of about 0 °C to 5 °C is further stirred for additional 1 hour to 3 hours, preferably for additional 2 hours under moderate stirring.

Eszopiclone particles may be recovered in step d) by the methods which are routine for a skilled person, such as filtration through funnels, canvas, industrial filters drawn by vacuum or over pressure, or centrifugation or decantation.

In one option isolated crystals may be dried under reduced pressure at temperature between 35 °C to 45 °C, preferably at 40 °C.

A preferred embodiment of the present invention is a process for preparing a crystalline eszopiclone having particles in the shape of sticks comprising the following steps:
a) mixing eszopiclone with an organic solvent to obtain a slurry,
b) heating the slurry of step a) to reflux to obtain a clear solution,
c) gradually cooling the solution to the temperature of 0 °C to 5 °C over about 2 hours to about 20 hours, preferably over about 4 hours to about 14 hours, more preferably over about 8 hours, under stirring at 50 rpm to 200 rpm to obtain precipitate,
c1) optionally stirring precipitate obtained in step c) at 50 rpm to 200 rpm for additional 1 hour to 3 hours, preferably for additional 2 hours,
d) recovering eszopiclone particles,
wherein an organic solvent is selected from the group consisting of C₁-C₆ alcohols, C₁-C₆ alkyl acetates, C₁-C₆ alkyl ketones and a mixture of C₁-C₆ alcohols and acetone.

Another preferred embodiment of the present invention is a process for preparing a crystalline eszopiclone having particles in the shape of sticks comprising the following steps:
a) mixing eszopiclone with an organic solvent to obtain a slurry,
b) heating the slurry of step a) to reflux to obtain a clear solution,
c1) gradually cooling to the temperature of 20 °C to 25 °C over about 1 hour to about 10 hours, preferably over about 2 hour to about 7 hours, more preferably over about 4 hours, under stirring at 50 rpm to 200 rpm,
c2) gradually cooling to the temperature of 0 °C to 5 °C over about 1 hour to about 10 hours, preferably over about 2 hours to about 7 hours, more preferably over about 4 hours, under stirring at 50 rpm to 200 rpm to obtain precipitate,
c3) optionally, stirring precipitate obtained in step c2) at 50 rpm to 200 rpm for additional 1 hour to 3 hours, preferably for additional 2 hours,
d) recovering eszopiclone particles,
wherein an organic solvent is selected from the group consisting of C₁-C₆ alcohols, C₁-C₆ alkyl acetates, C₁-C₆ alkyl ketones and a mixture of C₁-C₆ alcohols and acetone.

Another object of the present invention is use of a crystalline eszopiclone having particles in the shape of sticks according to the present invention for the preparation of pharmaceutical composition, preferably tablets.

Another object of the present invention is a pharmaceutical composition comprising a crystalline eszopiclone having particles in the shape of sticks according to the present invention. Preferred pharmaceutical compositions comprising eszopiclone are disclosed by EP 1997482 A1. Pharmaceutical compositions may be prepared by the methods well known in the field of the pharmaceutical technology. Preferably, the pharmaceutical compositions may be prepared according to the processes as disclosed by EP 1997482 A1.

In another embodiment the present invention relates to use of the pharmaceutical composition comprising a crystalline eszopiclone having particles in the shape of sticks according to the present invention for the preparation of a medicament with sedative and hypnotic properties.

### Brief description of the drawings

Figure 1 shows a SEM micrograph of eszopiclone crystallized from 2-propanol with stirring (Example 1) (magnified: x100, bar 100 µm)
Figure 2 shows a SEM micrograph of eszopiclone crystallized from ethyl acetate with stirring (Example 2) (magnified: x100, bar 100 µm)
Figure 3 shows a SEM micrograph of eszopiclone crystallized from 96% ethanol without stirring (Example 3) (magnified: x50, bar 100 µm)
Figure 4 shows a SEM micrograph of eszopiclone crystallized from 2-propanol without stirring (Example 4) (magnified: x100, bar 100 µm)
Figure 5 shows a SEM micrograph of eszopiclone crystallized from 2-propanol/Ethyl acetate (7/1) without stirring (Example 5) (magnified: x100, bar 100 µm)
Figure 6 shows a SEM micrograph of eszopiclone crystallized from ethyl acetate without stirring (Example 6) (magnified: x100, bar 100 µm)
Figure 7 shows a SEM micrograph of eszopiclone crystallized from isopropyl acetate without stirring (Example 7) (magnified: x100, bar 100 µm)
Figure 8 shows a SEM micrograph of eszopiclone crystallized from isobutyl acetate without stirring (Example 8) (magnified: x100, bar 100 µm)
Figure 9 shows a SEM micrograph of eszopiclone crystallized from 2-butanol without stirring (Example 9) (magnified: x100, bar 100 µm)
Figure 10 shows a SEM micrograph of eszopiclone crystallized from tetrahydrofuran without stirring (Example 10) (magnified: x100, bar 100 µm)
Figure 11 shows a SEM micrograph of eszopiclone crystallized from acetonitrile with stirring (Example11) (magnified: x100, bar 100 µm)
Figure 12 shows a SEM micrograph of eszopiclone crystallized from methyl ethyl ketone without stirring (Example 12) (magnified: x100, bar 100 µm)
Figure 13 shows a SEM micrograph of eszopiclone crystallized from methyl iso-butyl ketone without stirring (Example 13) (magnified: x100, bar 100 µm)

The following examples illustrate the invention, but do not limit it in any way.

### Example 1

A 2 L glass reactor equipped with a mechanical stirrer, condenser and thermometer was charged with eszopiclone (30 g, 0.08 mol) and 1200 mL of 2-propanol. The slurry was heated under reflux, resulting solution was cooled to 20 °C in 4 hours under stirring at 170 rpm and then to 0 °C in 4 hours under stirring at 170 rpm. Precipitated crystals were isolated by filtration and dried under reduced pressure at 40 °C for 17 hours to yield 27 g (89 %) of crystallized Eszopiclone with particles in the shape of sticks having a particle size distribution, d(0.9) = about 418 microns, d(0.5) = about 195 microns.

### Example 2

A 10 L glass reactor equipped with a mechanical stirrer, condenser and thermometer was charged with eszopiclone (242 g, 0.62 mol) and 6700 mL of Ethyl acetate. The slurry was heated under reflux, resulting solution was cooled to 23 °C in 4 hours under stirring at 100 rpm and then to 0 °C in 4 hours under stirring at 100 rpm. Precipitated crystals were held at 0 °C and under stirring at 100 rpm for additional 2 hours before they were isolated by filtration and dried under reduced pressure at 40 °C for 12 hours to yield 208 g (86 %) of crystallized S-zopiclone with particles in the shape of sticks having a particle size distribution, d(0.9) = about 403 microns, d(0.5) = about 145 microns.

### Example 3 - Comparative example

Eszopiclone (2 g, 5.1 mmol) in 60 mL of 96 % ethanol was heated under reflux, resulting solution was filtered and cooled to 3 °C in 1 hour 30 minutes without stirring. Precipitated crystals were held at 0-3 °C for additional 2 hours before they were isolated by filtration and dried under reduced pressure at 40 °C for 18 hours to yield 1.8 g (89 %) of crystallized eszopiclone with particles in the shape of tiles having a particle size distribution, d(0.9) = about 548 microns, d(0.5) = about 242 microns.

### Example 4 - Comparative example

Eszopiclone (250 g, 0.64 mol) in 12.5 L of 2-propanol was heated under reflux, resulting solution was filtered and cooled to 23 °C in 6 hours without stirring and then to 0-3 °C in 4 hours. Precipitated crystals were held at 0-3 °C for additional 14 hours before they were isolated by filtration and dried under reduced pressure at 40 °C for 21 hours to yield 242 g (97 %) of crystallized eszopiclone with particles in the shape of tiles having a particle size distribution, d(0.9) = about 385 microns, d(0.5) = about 167 microns.

### Example 5 - Comparative example

Eszopiclone (39 g, 0.10 mol) in 320 mL of Ethyl acetate and 2 L of 2-propanol was heated under reflux, resulting solution was filtered and cooled to 23 °C in 4 hours without stirring and then to 0-3 °C in 2 hours. Precipitated crystals were isolated by filtration, washed with 100mL of cold 2-propanol and dried under reduced pressure at 40 °C for 14 hours to yield 33 g (85 %) of crystallized eszopiclone with particles in the shape of tiles having a particle size distribution, d(0.9) = about 310 microns, d(0.5) = about 124 microns.

### Example 6 - Comparative example

Eszopiclone (35 g, 0.09 mol) in 930 mL of Ethyl acetate was heated under reflux, resulting solution was cooled to 35 °C in 1 hours 30 minutes under slow stirring with magnetic bar and then to 0 °C in 2 hours without stirring. Precipitated crystals were held at 0 °C for additional 16 hours before they were isolated by filtration, washed with 20 mL of Ethyl acetate and dried under reduced pressure at 40 °C for 3 hours to yield 25 g (71 %) of crystallized eszopiclone with particles representing a mixture of both shapes; tiles and few sticks, having a particle size distribution, d(0.9) = about 242 microns, d(0.5) = about 36 microns.

### Example 7 - Comparative example

Eszopiclone (4 g, 0.01 mol) in 230 mL of isopropyl acetate was heated under reflux, resulting solution was filtered and cooled to 26 °C in 2 hours without stirring and then to 0-3 °C in 2 hours. Precipitated crystals were isolated by filtration and dried under reduced pressure at 40 °C for 7 hours to yield 3.4 g (85 %) of crystallized eszopiclone with particles representing a mixture of both shapes; tiles and few sticks, having a particle size distribution, d(0.9) = about 379 microns, d(0.5) = about 115 microns.

### Example 8 - Comparative example

Eszopiclone (3 g, 7.7 mmol) in 85 mL of isobutyl acetate was heated under reflux, resulting solution was filtered and crystals precipitated instantly. The slurry was cooled to 25 °C in 30 minutes without stirring and then to 5 °C in 1 hour and 30 minutes. Crystals were isolated by filtration and dried under reduced pressure at 40 °C for 20 hours to yield 2.7 g (92 %) of crystallized eszopiclone with particles representing a mixture of both shapes; tiles and few sticks having a particle size distribution, d(0.9) = about 250 microns, d(0.5) = about 73 microns.

### Example 9 - Comparative example

Eszopiclone (2 g, 5.1 mmol) in 40 mL of 2-butanol was heated under reflux, resulting solution was filtered and cooled gradually to 0 °C in 2 hours without stirring. Precipitated crystals were held at - 18 °C for additional 20 hours before they were isolated by filtration and dried under reduced pressure at 40 °C for 4 hours to yield 1.9 g (95 %) of crystallized eszopiclone with particles in the shape of lumps and having a particle size distribution, d(0.9) = about 107 microns, d(0.5) = about 49 microns.

### Example 10 - Comparative example

Eszopiclone (2 g, 5.1 mmol) in 24 mL of tetrahydrofuran was heated under reflux, resulting solution was filtered and cooled gradually to 0 °C in 2 hours without stirring. Precipitated crystals were held at - 18 °C for additional 20 hours before they were isolated by filtration and dried under reduced pressure at 40 °C for 4 hours to yield 1.4 g (970 %) of crystallized eszopiclone with particles in the shape of lumps and having a particle size distribution, d(0.9) = about 656 microns, d(0.5) = about 375 microns.

### Example 11 - Comparative example

Eszopiclone (43.7 g, 0.11 mol) in 405 mL of acetonitrile was heated under reflux, resulting solution was filtered and cooled gradually to 25 °C in 1 hours under stirring. The slurry was then cooled to 0 °C in additional hour without stirring. Precipitated crystals were isolated by filtration and dried under reduced pressure at 40 °C for 20 hours to yield 34.7 g (79 %) of crystallized eszopiclone with particles in the shape of tiles gathered in lumps and having a particle size distribution, d(0.9) = about 179 microns, d(0.5) = about 64 microns.

### Example 12 - Comparative example

Eszopiclone (19.2 g, 0.05 mol) in 500 mL of methyl ethyl ketone was heated under reflux, resulting solution was cooled to 25 °C in 3 hours without stirring and then to 4 °C in 1 hour and 20 minutes. Precipitated crystals were held at -18 °C overnight before they were isolated by filtration and dried under reduced pressure at 40 °C for 4 hours to yield 14.6 g (76 %) of crystallized eszopiclone with a mixture of particles in the shape of small and larger tiles having a particle size distribution, d(0.9) = about 278 microns, d(0.5) = about 36 microns.

### Example 13 - Comparative example

Eszopiclone (2 g, 5.1 mmol) in 30 mL of methyl iso-butyl ketone was heated under reflux, resulting solution was filtered and cooled to 25 °C in half an hour without stirring and then to 0 °C in 1 hour and 40 minutes. Precipitated crystals were held at -18 °C for 3 hours before they were isolated by filtration and dried under reduced pressure at 40 °C for 23 hours to yield 1.7 g (85 %) of crystallized eszopiclone with a mixture of particles in the shape of small and larger needles having a particle size distribution, d(0.9) = about 250 microns, d(0.5) = about 73 microns.

### Example 14

### Analytical methods

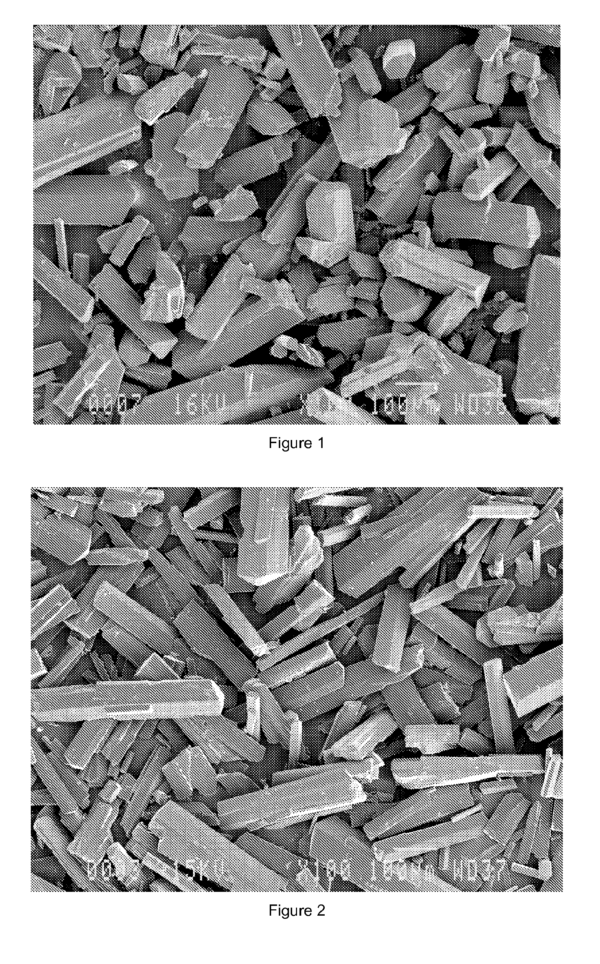

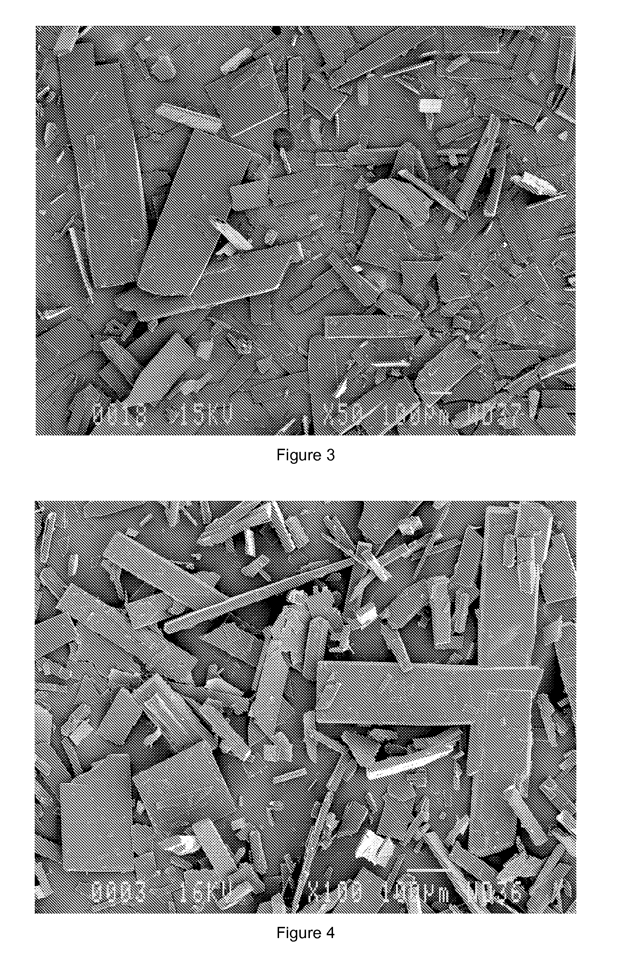

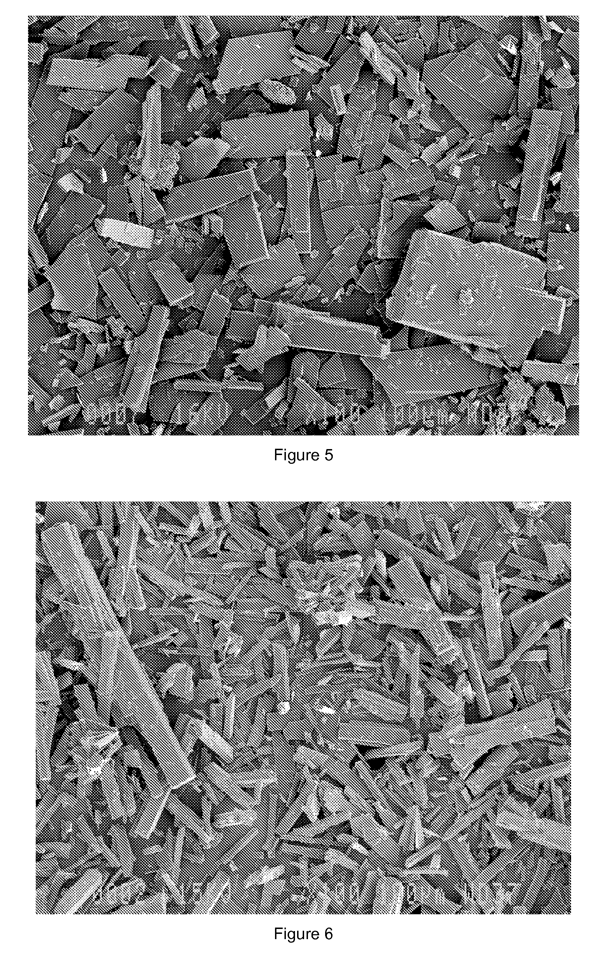

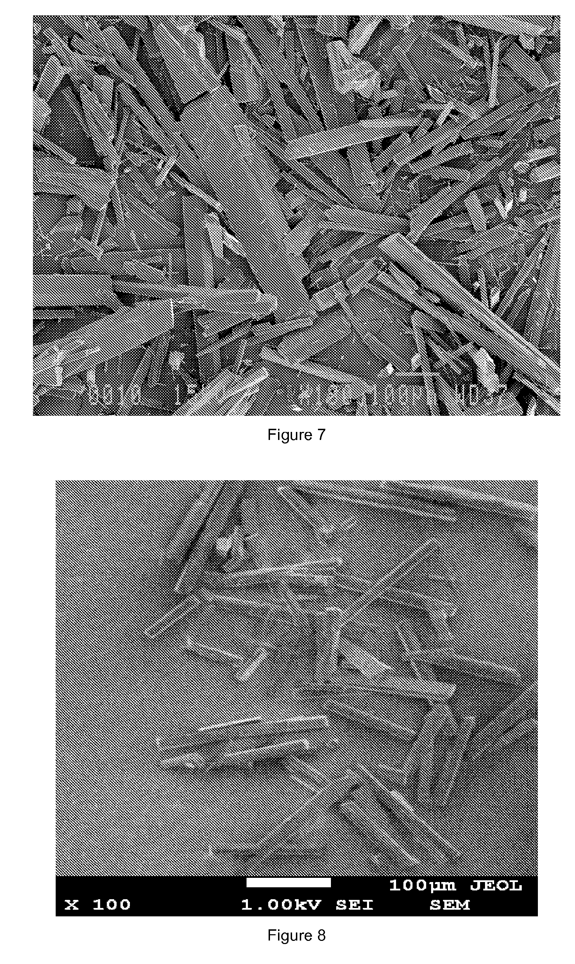

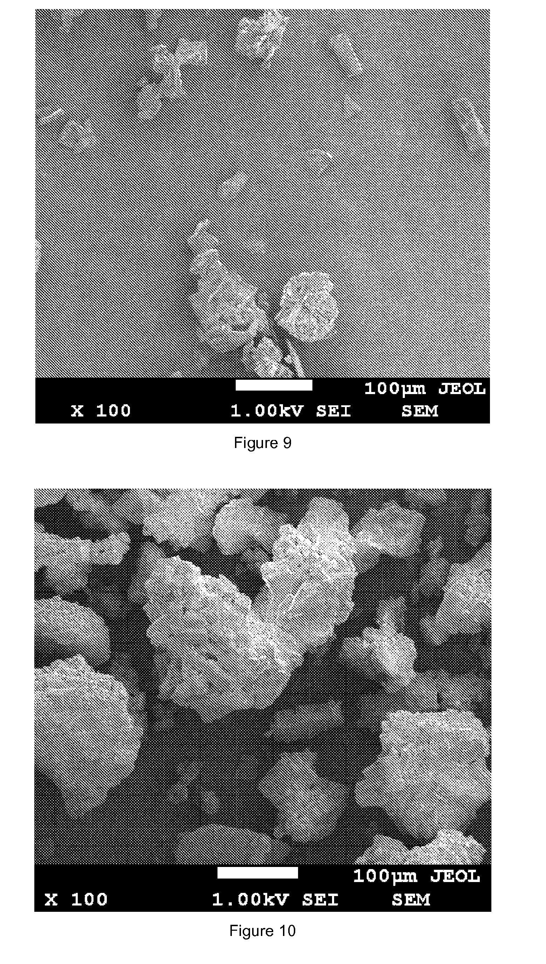

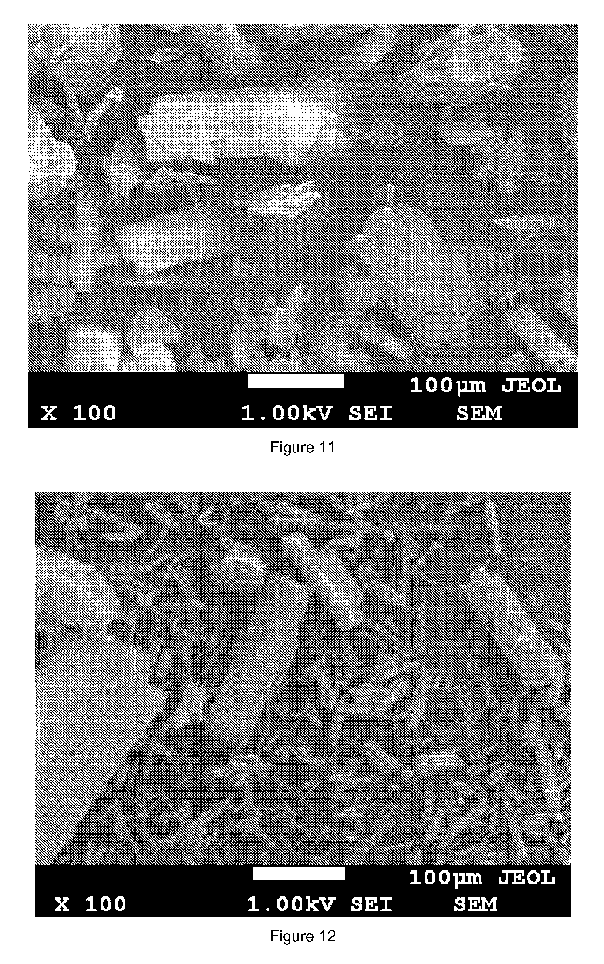

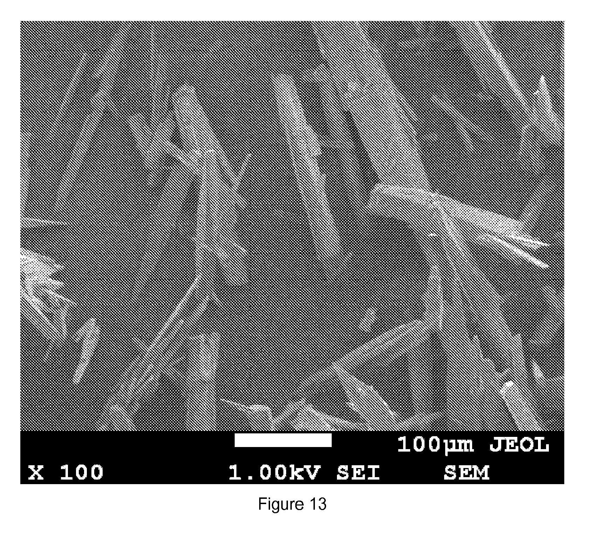

SEM photographs (Figures 1 to 8) of the powders were taken using a JEOL JXA 840A Scanning Electron Microscope with an accelerating voltage of 15 or 16 kV. SEM photographs (Figures 9 to 13) of the powders were taken using a JEOL FE JSM-7001 F Scanning Electron Microscope with an accelerating voltage of 1 kV.

Particle size distribution of eszopiclone was characterized by laser diffraction (Malvern Mastersizer S). Sample cell was small volume sample dispersion cell MS1, presentation was 3NHE, solvent was hexane, stirrer speed was 2000 rpm. The following procedure was used: Fill the sample cell with hexane, add 100 mg of sodium bis (2-ethylhexyl) sulfosuccinate, align the sizer and measure the background. Add sample into the sample cell until proper obscuration is achieved (10-30%). Analyze when the signal from detectors is stable.

## Claims

1. A crystalline eszopiclone having particles with average ratio of width and length between 1:2 and 1:15 and average ratio of thickness and width between 1:1 and 1:5.

2. A crystalline eszopiclone according to claim 1 having a particle size distribution as determined by volume by laser-diffraction method of d(0.9) is between 200 µm and 800 µm.

3. A crystalline eszopiclone particles according to claim 1 having a particle size distribution as determined by volume by laser-diffraction method of d(0.9) is between 350 µm and 450 µm.

4. A crystalline eszopiclone particles according to claim 3 having a particle size distribution as determined by volume by laser-diffraction method of d(0.5) is between 100 µm and 250 µm.

5. A process for preparing a crystalline eszopiclone particles comprising the following steps:
a) mixing eszopiclone with an organic solvent to obtain a slurry,
b) heating the slurry of step a) to reflux to obtain a clear solution,
c) cooling the solution under stirring at 50 rpm to 200 rpm,
d) recovering eszopiclone particles,
wherein an organic solvent is selected from the group consisting of C₁-C₆ alcohols, C₁-C₆ alkyl acetates, C₁-C₆ alkyl ketones and a mixture of C₁-C₆ alcohols and acetone.

6. A process according to claim 5, wherein a cooling in step c) is gradually cooling to the temperature of 5 °C to 0 °C over 2 hour to 20 hours.

7. A process according to claim 5, wherein a cooling in step c) is a two stage cooling comprising the following sub-steps of:
c1) gradually cooling to the temperature of 20 °C to 25 °C over 1 hour to 10 hours,
c2) gradually cooling to the temperature of 0 °C to 5 °C over 1 hour to 10 hours.

8. A process according to any one of claims 5 to 7, wherein precipitate obtained in step c) is stirred at 50 rpm to 200 rpm for additional 1 hour to 3 hours.

9. Use of a crystalline eszopiclone according to any one of claims 1 to 4 for the preparation of pharmaceutical composition.

10. A pharmaceutical composition comprising a crystalline eszopiclone according to any one of claims 1 to 4.
